(19) Europäisches Patentamt

European Patent Office

Office européen des brevets

(11) Publication number: **0 581 353 A1**

## (12) EUROPEAN PATENT APPLICATION

(21) Application number: **93201959.9**

(22) Date of filing: **05.07.93**

(51) Int. Cl.5: **C12N 5/10, C12P 21/08, A61K 39/42**

The applicant has subsequently filed a sequence listing and declared, that it includes no new matter.

(30) Priority: **03.07.92 EP 92202032**

(43) Date of publication of application:
**02.02.94 Bulletin 94/05**

(84) Designated Contracting States:
**AT BE CH DE DK ES FR GB GR IE IT LI LU MC NL PT SE**

(71) Applicant: **DE STAAT DER NEDERLANDEN VERTEGENWOORDIGD DOOR DE MINISTER VAN WELZIJN, VOLKSGEZONDHEID EN**

**CULTUUR**
**P.O. Box 5406**
**NL-2280 HK Rijswijk(NL)**

(72) Inventor: **Osterhaus, Albertus Dominicus Marcellinus E.**
**Dr. Breveestraat 16**
**NL-3981 CH Bunnik(NL)**

(74) Representative: **de Bruijn, Leendert C. et al**
**Nederlandsch Octrooibureau**
**Scheveningseweg 82**
**P.O. Box 29720**
**NL-2502 LS Den Haag (NL)**

(54) **Monoclonal antibodies to HiV-1 gp120, cell lines producing them and corresponding epitope structures.**

㊄ The invention provides monoclonal antibodies to HIV-1, which react with a HIV-1 gp120 glycoprotein or first variant thereof wherein the aminoacids Asn-88, Lys-117, Asn-262 and Tyr-435 are present, and exhibit an at least 50% reduced reaction with a second variant of said glycoprotein which differs from said glycoprotein or first variant thereof in that one or more of said aminoacids has been deleted or mutated. Furthermore, the invention provides compositions for passive and active immunization based on these antibodies and on anti-idiotype antibodies, respectively; as well as cell lines and processes for producing these antibodies.

Fig-3.1

The present invention relates to monoclonal antibodies to HIV-1, and to vaccines based on these antibodies.

Background of the invention

The human immune response to HIV-1 is believed to play a major role in the pathogenesis of the HIV-1 infection, and in the relatively long delay in the development of clinical symptoms after infection (Ascher and Sheppard, 1988; Robinson *et al*. 1991; Fung *et al*. 1992). Therefore the study of both B and T cell mediated HIV-1 specific immune response in seropositive individuals is of major importance for the development of active and passive immunization strategies. Sera from HIV-1 infected individuals have been shown to neutralize diverse strains of HIV-1 originating from Europe, the United States and Africa (Weiss *et al*. 1986). Several studies in which virus neutralizing (VN) antibodies were passively transferred, indicated that antiviral antibodies play a major role in the protection from lentivirus infections (Karpas *et al*. 1990; Putkonen *et al*. 1992; Prince *et al*. 1991; Emini *et al*. 1992). Different approaches have been considered to identify epitopes involved in the neutralization of HIV-1. Among these the so-called pepscan method has been quite successful. With this method, using partially overlapping peptides representing the complete sequence of gp120 on the one hand, and monoclonal and polyclonal antibody preparations on the other hand, the principal neutralizing domain (PND) was identified within the third variable region (V3 loop) of gp120 (Goudsmit *et al*. 1988a; Goudsmit *et al*. 1988b). VN mouse monoclonal antibodies (mabs) specific for the V3 loop, exhibit a relatively high degree of strain specificity. However, some of the mouse mabs of this specificity do recognize viruses which are antigenically quite distinct. Part of the core of the epitope recognized by most of these antibodies is the relatively conserved $\beta$-turn at the tip of the loop (GPG) (Broliden *et al*. 1992; LaRosa *et al*. 1990; Langedijk *et al*. 1991; Ohno *et al*. 1991; Laman e*t al*. 1992). Broadly reactive VN antibodies not reactive with the V3 loop, which were found in the sera of seropositive individuals, appeared to recognize a conformation dependent epitope which could not be identified with the pepscan method. These antibodies inhibit the binding of gp120 to CD4 and are responsible for much of the cross reactivity in human sera (Kang *et al*. 1991; Steimer *et al*. 1991). Sofar a limited number of human mabs specific for the antigenic site have been derived from North American seropositive individuals and rodent mabs have been generated against virus strains which originate from the USA (Robinson *et al*. 1990; Posner *et al*. 1991; Fung *et al*. 1992; Cordell *et al*. 1991; Ho *et al*. 1991).

Summary of the invention

The present invention deals with the generation of VN (virus neutralizing) human mabs. Such mabs can be produced by Epstein Barr virus (EBV) transformation of peripheral blood mononuclear cells (PBMC's) from seropositive individuals. The characterization of these antibodies and the corresponding epitopes are also presented.

Four EBV transformed B cell lines were generated from the PBMC of two Dutch HIV-1 seropositive individuals, which produce human mabs that neutralize HIV-1. Three of these antibodies proved to be directed to a conserved conformation dependent site that overlaps with the CD4 binding site of gp120. The fourth was directed to the PND of gp120 and showed a much more restricted reactivity pattern.

Accordingly, the invention relates to human or animal monoclonal antibodies to HIV-1, characterised in that they react with a HIV-1 gp120 glycoprotein or first variant thereof wherein the aminoacids Asn-88, Lys-117, Asn-262 and Tyr-435 are present, and exhibit an at least 50% reduced reaction with a second variant of said glycoprotein which differs from said glycoprotein or first variant thereof in that one of said aminoacids has been deleted or mutated.

In particular, the invention relates to such monoclonal antibodies, which react with said glycoprotein gp120 or first variant thereof wherein said aminoacids Asn-88, Lys-117, Asn-262 and Tyr-435 are present, and which exhibit an at least 75% reduced reaction with a second variant of said glycoprotein wherein aminoacid Asn-88 or Asn-262 has been deleted or mutated.

The invention relates more specifically to human mabs designated GP13, GP44 and GP68, which at the same time are broadly reactive and HIV-1 specific, since on the one hand they react in an immunostaining assay with a wide variety of HIV-1 strains originating from Europe, the USA and Africa and on the other hand they fail to recognize the envelope glycoproteins of a HIV-2 strain, SIV-mac and FIV in an ELISA.

The invention also relates to human cell lines that produce the antibodies to HIV-1, to a method of producing the antibodies, to preparations for passive immunotherapy and to vaccines containing the corresponding epitope structures.

The invention is also concerned with aminoacid sequences corresponding to immunodeterminant regions of anti-HIV-1 antibodies and with nucleotide sequences encoding these aminoacids.

Furthermore, the invention is concerned with antibodies (idiotypic or idiotype derived structures) directed to a HIV-1 antibody as mentioned above, in which the principle of anti-idiotypic immunization for the induction of antiviral immunity is used (cf. "Idiotype Networks", in: Biology and Medicine, Ed. A. Osterhaus and F. Uytdehaag, Excerpta Medica, Amsterdam/New York/Oxford, 1990).

Detailed description of the invention

It has been found according to the invention that monoclonal antibodies raised against HIV-1 are specific to HIV-1 and also show a broad reactivity to geographic variants of HIV-1 when they exhibit a particular recognition pattern to the HIV-1 glycoprotein gp120. The aminoacid sequence of gp120 of HIV-1 strain HXB2 is known (Meyers *et al*, 1989). Important gp120 aminoacids according to the invention are aminoacids Asn-88, Lys-117, Asn-262 and Tyr-435. Reaction with gp120 or a variant thereof containing these aminoacids and reduced reaction with a variant wherein either of these aminoacids is absent as a result of a deletion or mutation, is indicative of antibodies with the required specificity. Reduced reaction is understood to mean a reduced binding reaction in known immunoassays used to characterize the specificity of antibodies to permutated glycoproteins. A reduction of 50 % is considered as being sufficiently distinctive. In particular, a mab showing a 75 % reduced reaction to a gp120 variant lacking either Asn-88 or Asn-262 has a high probability of being effective for developing passive immunization to HIV-1, and its anti-idiotypic counterpart for active immunization. In addition to the distinction by the four aminoacids mentioned above, a distinction can be made using mutations of the aminoacids Ser-256, Thr-257, Asp-368, Glu-370 or Tyr-384.

A gp120 variant wherein any of the aminoacids identified above is deleted or mutated is preferably a variant wherein such aminoacid is substituted by an aminoacid which is different in size, charge, hydrophilicity, acidity or another relevant parameter.

Specific human monoclonal antibodies of the type referred to above are those that are designated GP13, GP44 or GP68. The aminoacid sequences of the variable regions of these mabs are presented in the sequence listings included below.

The VN pattern of one of the human mabs (GP44) is more restricted (Table 4), which may be due to a relatively low affinity. Our human mabs GP13 and GP68 exhibited a relatively broad VN activity, which included reactivity with HIV-1 strains from different continents. In general a broad reactivity is not observed with human mabs directed against the V3 loop (Gorny *et al*. 1991). Rodent mabs against the V3 loop, however, did exhibit a somewhat broader specificity in VN assays. This is probably due to both qualitative and quantitative differences in the recognition of aminoacid combination (Langedijk *et al*. 1991; Broliden *et al*. 1992). The CD4 binding site specific human mabs were tested in an ELISA for their reactivities with a previously described set of mutant gp120 molecules, which were all mutated for one aminoacid as compared to wild type HXBc2. These mutant gp120 molecules had previously been tested for their precursor processing, cell association and CD4 binding activities (Olshevsky *et al*. 1990) and also for their ability to bind to a previously described human mab F105 which has a similar specificity (Posner *et al*. 1991). Comparison of the specificities of these human mabs on the basis of published data is difficult to achieve, since the assay systems used by the respective labs vary considerably. The reactivity patterns of the different human mabs however, indicate that the three newly generated CD4 binding site specific human mabs have quite similar but still distinct specificities. At least four aminoacid (256, 257, 368, 370) seem to be at the core of the antigenic site, as judged from the loss of reactivity with CD4 and all the presently tested human mabs. Aminoacids 88, 117, 262 and 435 were newly identified as important for binding of one or more of the human mabs generated in these studies. It is interesting to note that changes in aminoacids 427 and 457 interfere with the binding of CD4, but not with any of the known human mabs.

The core of the epitope recognized by human mab MN215 was determined with the pepscan method. In the first series of experiments the HIV-1 MN V3 loop sequence was used to generate peptides varying in length from 8 to 16 aminoacids with one aminoacid overlap. With these peptides the minimal epitope recognized was 15 aminoacid in length and included aa at the N- and C-terminal side of the GPG $\beta$-turn. The minimal epitope recognized in this assay by human mab MN215 on the MN sequence is the longest found sofar. When peptides were generated with a sequence more related to the sequence found in recombinant envelopes from Dutch origin (Groenink *et al*. 1991), the core of the epitope was 9 aminoacids in length and located only at the C-terminal side of the GPG $\beta$-turn of the V3 loop. These data seem to indicate that although human mab MN215 does neutralise the HIV-1 MN strain it does not have its highest affinity for the MN sequence. As this human mab also neutralizes the HIV-1 SF2 strain it may be stated that certain human mabs specific for the V3 loop may have a cross-reactivity with HIV-1 strains that are only distantly related.

Generation of human mabs from seropositive individuals of different geographical areas is important for a better understanding of both the variability of the virus and in the immune response they elicit. This is also stressed by the differences in reactivity found with our panel of human mabs and with several other mabs directed against epitopes of the same antigenic site on gp120. The practical use of human mabs with broad HIV-1 VN activity will not be limited to the identification of B cell epitopes relevant for vaccine development based on internal images of the epitope of the antibodies, but may also be exploited in the field of passive immunization (Emini *et al*. 1992). Especially the use of high titered human mabs with broad HIV-1 VN activity as developed in the course of these studies, may be of particular value for this purpose.

The monoclonal antibodies according to the invention are further characterised by their partial aminoacid sequence. Partial nucleotide sequences and corresponding aminoacid sequences of the mab's GP13, GP44 and GP68 are given in the accompanying SEQ ID NO's 4, 5 and 6 respectively (variable regions of the light chain: GP13 $V_x$IV, GP44 $V_\lambda$2 and GP68 $V_x$1) and SEQ ID NO's 7, 8 and 9 respectively (variable regions of the heavy chain: GP13 $V_H$V, GP44 $V_H$I and GP68 $V_H$I). The antibodies of the invention can comprise an aminoacid sequence in which at least five aminoacids are situated in the same relative position as the aminoacid sequences represented in any one of the SEQ ID NO's 4-9. Particularly, they comprise at least a sequence of five aminoacids corresponding to five consecutive aminoacids from the sequences represented in SEQ ID NO's 4-9, either within the Complementarity Determining Regions (CDR I, II and III) identified in SEQ ID NO's 4-9, or within the framework regions, such as between CDR II and CDR III, or within the J segments, or combinations of five or more aminoacids from these regions. More specifically, they comprise at least three amino acids of any one of the Complementarity Determining Regions. The antibodies may comprise the entire sequences of the variable regions of SEQ ID 4 and/or 7, or 5 and/or 8, or 6 and/or 9, but they may also contain other aminoacids within the variable regions than those given in these SEQ ID's, especially if these other aminoacids do not significantly alter the tertiary structure of the polypeptide. In addition the antibodies may contain the constant regions of according to those of GP13, GP44 or GP68. Instead they may also comprise other constant regions, which still allow the biological function of the antibodies to be performed, as is known to a person skilled in the art.

The nucleotide sequences corresponding to the variable regions of the light chains and heavy chains of the monoclonal antibodies GP13, GP44 and GP68 were determined by art-known methods involving isolating messenger RNA from cell lines producing the antibodies and sequencing cDNA of the properly probed mRNA. These nucleotide sequences can be used in a manner known in the art of recombinant technology, e.g. for producing active antibody fragments and for producing modified fragments with altered activity. The invention also encompasses such nucleotide sequences having at least 15 consecutive bases corresponding to the nucleotide sequences given in SEQ ID NO's 4-9.

## MATERIALS AND METHODS

### Cell lines and virus strains

Human HELA CD4$^+$ cells were provided by P. Maddon (Maddon *et al*. 1986) and were maintained in Dulbecco's modified Eagles medium and 5% fetal calf serum (FCS). Human lymphoid cell lines C8166 (Salahuddin *et al*. 1983) and H9 (Popovic *et al*. 1984) were maintained in RPMI 1640 and 10% FCS. The following virus strains were used for the immunostaining and virus neutralisation: IIIB (LAI isolate Wain-Hobson *et al*. 1991), RF and MN (Popovic *et al*. 1984) were obtained from R.C. Gallo, SF2 was a gift of J. Levy (Levy *et al*. 1985), NY-5/LAV-1 (Adachi *et al*. 1986) and Z34, Z84 and Z129 were provided by M. Martin (Weiss *et al*. 1986), GL-1 and GL-3 were a gift from G. Farrar (Cordell *et al*. 1991), U455 was supplied by J. Oram (Oram *et al*. 1990), CLB-4 was isolated at the Chester Beatty Laboratories in collaboration with M. Popovic (Weiss *et al*. 1986). CBL-22 was also isolated at the Chester Beatty Laboratories from a HIV-2 infected individual in the Gambia in collaboration with H, Whille of the MRC laboratories. The HIV-2 strain LAV-2 was isolated by F. Clevel (Clevel *et al*. 1986).

### Generation of human B cell clones and human mabs

PBMC's were isolated on Ficoll gradients from heparinised blood of two HIV-1 seropositive asymptomatic adult males from the Amsterdam cohort as previously described (Teeuwsen *et al*. 1990). These PBMC's were enriched for B cells by a panning technique (Wysocki and Sato, 1980). In short T cells were adsorbed to a culture flask coated with a CD3 specific monoclonal antibody (RIV9) in a one hour 37°C

EP 0 581 353 A1

incubation. Non-adherent cells were incubated with Epstein Barr virus (EBV) derived from the supernatant of the EBV producing cell line B95-8 as previously described (Steinitz *et al.* 1977). After EBV transformation the cells were plated in 96 wells round-bottomed culture plates (Greiner, 650180) at 500-1000 cells per well with $10^5$ gamma irradiated (1800 rad) allogenic PBMC's from a seronegative blood donor. Cultures were maintained in RPMI 1640 (GIBCO, 041-02400 M) supplemented with 10% FCS (Bocknek ltd., 3008-502), 7 $\mu$g/ml phytohaemagglutinin (PHA-M, Boehringer-Mannheim),100 IU/ml penicillin, 100 $\mu$g/ml streptomycin, 2 mM L glutamine and $2 \times 10^{-5}$ M $\beta$-mercaptoethanol. Cells in wells that were three times above background $OD_{450}$ in the screening systems used, were subcloned at concentrations which resulted in less than 30% growth rate. The subclass determination was performed in ELISA with subclass specific mabs (Sera-Lab, MAS 265-268 p) as described previously (Teeuwsen *et al.* 1990). The amount of Human mab produced by the B cell lines generated was determined by the amount of Human mab obtained from a protein A column using 500 ml. of culture supernatant as starting material and was expressed as $\mu$g protein per ml supernatant (Bradford, 1976).

**Screening systems for detection of gp120 specific human mabs**

For the identification of HIV-1 gp120/gp160 specific antibodies in culture supernatants, two different ELISA's were used. In the first ELISA microtiter plates (Costar, High Binding, flat bottomed) were coated with Concanavaline A (ConA, Farmacia, Uppsala, Sweden) (10 $\mu$g/well in 100 $\mu$l of phosphate buffered saline (PBS), 1 hour 37°C) and subsequently incubated with 75 ng/well recombinant HIV-1 IIIB gp120/gp160 produced in a baculovirus expression system (American Biotechnologies Inc., Cambridge Mass. USA, catalog nr. 24001 and 12001). Both recombinant glycoproteins have been shown to bind CD4 (Moore *et al.* 1990). In the second ELISA the microtiter plates were coated with peptides representing parts of the V3 loop of different HIV-1 strains (American Biotechnologies Inc., cat.nr. 623-630 010). After coating the wells were blocked overnight at 4°C with RPMI 1640 10% FCS 1:2 diluted with PBS. The wells were washed twice and subsequently incubated with culture supernatant (1:2 diluted with PBS, 1% Triton X-100) from the culture plates 30 to 40 days after initial cloning. After another washing step bound human antibody was detected with the isolated IgG fraction of goat anti human IgG labelled with horseradish peroxidase (HRP) (Sigma, cat.nr. A-8775). The plates were developed with tetramethylbenzidine-$H_2O_2$ and the reaction was stopped by the addition of 2 M $H_2SO_4$. The optical density at 450 nm was read in a Titertek Multiskan (Flow Laboratories). The human mabs were also tested in ELISA for reactivity with a SIV-mac cell lysate and HIV-2 gp105 (American Biotechnologies Inc., cat.nr. 15001) both bound to Con-A coated plates and on vaccinia recombinant FIV glycoprotein, produced in HELA cells, coated in acidic pH on Costar High binding flat bottomed microtiter plates (Siebelink *et al.* 1992). Binding of antigen to the ELISA plate was checked with monoclonal (HIV-1 gp120, HIV-1 gp160 and HIV-1 IIIB V3 loop peptide) or polyclonal human, cat or chimpanzee (HIV-2, SIV-mac glycoprotein, FIV gp160, HIV-1 MN V3 loop peptide) serum antibodies directed against the antigen coated.

**Inhibition ELISA**

The human mabs GP13 and GP68 generated in the course of this study were biotinylated with NHS-d-succinimidobiotin as recommended by the manufacturer (Sigma Chemie, Axel, the Netherlands). First the dilution resulting in the half maximum $OD_{450}$ (midpoint titre) of the biotinylated human mabs was determined on the ConA-gp120 ELISA. The ConA-gp120 plates were incubated for one hour with 50 $\mu$l of 7$\mu$g/ml antibody or 75 ng/well sCD4. As negative control a Human mab (IgG1) specific for HIV-1 gp41 was used (Teeuwsen *et al.* 1990). Then 50 $\mu$l of biotinylated Human mab was added resulting in the midpoint titre dilution. After one hour 37°C the ELISA was further developed with streptavidin conjugated HRP (Immunoselekt, GIBCO BRL) as previously described (Teeuwsen *et al.* 1990). These assays were performed in triplicate, given is the mean of these three samples. The conformational nature of the epitope was determined in an ELISA using nitrocellulose as binding bottom of the well (millititer™ HA filtration plate, Millipore). In this ELISA native and denatured (1% SDS, $\beta$-mercaptoethanol, 2 min. 90°C) baculovirus produced gp120 from the HIV-1 IIIB strain (American Biotechnologies Inc., cat.nr 12001) was coated onto the nitrocellulose bottom of the ELISA plate and the ELISA was further developed as described by the manufacturer. Both native gp120 and denatured gp120 did bind to the nitrocellulose bottom as demonstrated with serum of a HIV-1 infected individual.

5

### Radio-immunoprecipitation

For radioimmunoprecipitation (RIPA) HELA cells were metabolically labelled with [$^{35}$S] methionine and [$^{35}$S] cysteine (TRAN$^{35}$S label, ICN Biomedicals) twelve hours after infection with 10 PFU of recombinant vaccinia virus per cell. The four recombinants used contained envelope genes of both N.S.I. and S.I. envelope glycoproteins (A.C. Andeweg et al.). After labelling the cells the medium was reconstituted with medium containing unlabelled methionine and cysteine for five hours after which supernatant was collected. The immunoprecipitation was performed under denaturing (1% DOC, 1% Triton X-100) and non-denaturing conditions with polyclonal sheep anti gp120 serum (kindly provided by Dr. M. Page through the MRC AIDS reagent project) and the relevant human mabs. The precipitated proteins were analysed by Sodium Dodecyl Sulphate polyacrylamide gel electrophoresis (SDS-PAGE, 10% acrylamide) according to Laemmli (Laemmli, 1970) with a phosphor imager.

### ELISA with mutant GP120's

The ELISA used for the determination of the fine specificity of the CD4 binding site specific human mabs was performed with a standard assay developed by J. McKeating (Moore *et al*. 1989). In this assay 15 ng/ml of mutant gp120 (Olshevsky *et al*. 1990), as determined after binding to the ELISA plate, was coated to the ELISA plate with affinity purified polyclonal antibody raised in sheep against the HIV-1 carboxy terminus (D7324, Aalto bioreagents). The ELISA was developed with a amplification kit (Ampak, Novo Biolabs, England). The percentage reduction in binding is given as mean % from wild type from two independent assays and calculated according to the formula

$$\% = 100 x \left( \frac{OD450mutant - ODbackground}{OD450WT - ODbackground} \right)$$

### Pepscan

Pepscan was performed originally described by Geyssen and Meloen (Geyssen *et al*, 1984). In short, peptides were synthesized on polyethylene rods and tested with Human mab MN215 in an ELISA according to established procedures (Langedijk *et al*. 1991). A set of 8 to 16 aa (aminoacids) long peptides with all but one aa overlap was synthesized with sequences derived from the V3 loop from the MN strain (PNYNKRKRIHIGPGRAFYTTKNIIGTIRQ) (SEQ ID NO 1) (Meyers *et al*. 1989) and one sequence more related to the V3 loop sequences found in serum from donor #658 taken at seroconversion (NTRKSIHIGPGRAFYTTGEIID) (SEQ ID NO 2).

### Immunostaining

The immunostaining was performed on HELA/CD4 cells infected with HIV strains three days prior to staining. The method was adapted from that described by Chesbro and Wehrly (Chesbro and Wehrly 1988) and has been described in detail (Clapham *et al*. 1992). After one hour incubation at 37°C the cells were fixed with methanol/acetone (1:1) and then blocked. The wells were incubated with HIV specific antibody and then bound antibody was detected with $\beta$-galactosidase coupled polyclonal sheep anti human IgG (Sigma chemie, Axel, the Netherlands). A human mab was considered positive on a virus isolate if syncytia and a percentage of cells colour positive as compared to the negative control without specific human mab.

### Virus Neutralisation assay

The microtiter VN assay used in these studies has been described before (McKeating *et al*. 1989). In short 1000 TCID$_{50}$ in 40 $\mu$l of virus stock was preincubated with 10$\mu$l of serial dilutions of protein A column purified Human mab for one hour 37°C and then incubated with 100$\mu$l of 2x10$^5$/ml C8166 (H9 was also used for some assays) for one hour at 37°C. The cells were then cultured and after five days the wells were scored for syncytia. The VN capacity was tested using Protein A purified Human mab. Six HIV-1 strains (HIV-1 SF2, MN, IIIB, RF, CBL4, Z34) from different geographical areas and two HIV-2 strains (HIV-2 LAV-2, CBL-22) were used in this assay (Meyers *et al*. 1989).

**RESULTS**:

**Generation and characteristics of gp120 specific human mabs**

Screening of cloned EBV transformed PBMC from donors #1171 and #658 of the Amsterdam cohort yielded one B cell line from the first (EBV GP13) and two from the second donor (EBV GP44, EBV GP68) which produced human mabs reactive in an ELISA with baculovirus recombinant HIV-1 IIIB gp120 and gp160 (Table 1). Another B cell line from the second donor (EBV MN215) produced antibodies reactive in an ELISA with a 20 aa peptide corresponding to the V3 loop aa sequence of the MN strain (Meyers *et al*. 1989) (table 1). All four human mabs were of the IgG1 subclass as determined in ELISA. They produced approximately 10 $\mu$g/ml (Human mab GP13), 8 $\mu$g/ml (Human mab GP44), 4 $\mu$g/ml (Human mab GP68) and 1.2 $\mu$g/ml (Human mab MN215) for more than six months. To delineate the antigenic sites recognized by these four human mabs, we tested the capacity of these human mabs and sCD4, to inhibit the binding of biotin-conjugated human mabs GP13 and GP68 to HIV-1 IIIB gp120 in an inhibition ELISA. A previously characterized IgG1 Human mab (Teeuwsen *et al*. 1990) specific for gp41 (Human mab K14) was included as a negative control. Human mabs GP13, GP44 and GP68 showed inhibition percentages with both biotin conjugates between 10 and 60 %, whereas human mabs MN215 and K14 showed inhibition lower than five percent. sCD4 showed 42% and 31% inhibition with these conjugates respectively (table 2). These data show that the human mabs GP13, GP44 and GP68 recognize the same antigenic site or sites in each others proximity which are located within or topographically near the CD4 binding site of gp120.

**Specificity of human mabs GP13, GP44 and GP68**

Human mabs GP13, GP44 and GP68 did not recognize the 22 aa peptides representing part of the V3 loops of HIV-1 HXB2 and HIV-1 MN (table 1) nor any of the other peptides tested including one involved in the CD4 binding site of HIV-1 (Lasky *et al*. 1987; Dowbenko *et al*, 1988) (data not shown). These three human mabs also failed to recognize envelope glycoproteins of HIV-2, SIV-mac, and FIV in an ELISA (table 1). To further determine the degree of conservation of the epitopes recognized by these three human mabs, they were tested in the immunostaining assay with virus strains from different geographical areas. Twelve HIV-1 strains tested were recognized by these human mabs in this assay whereas two HIV-2 strains tested were not recognized (table 3).

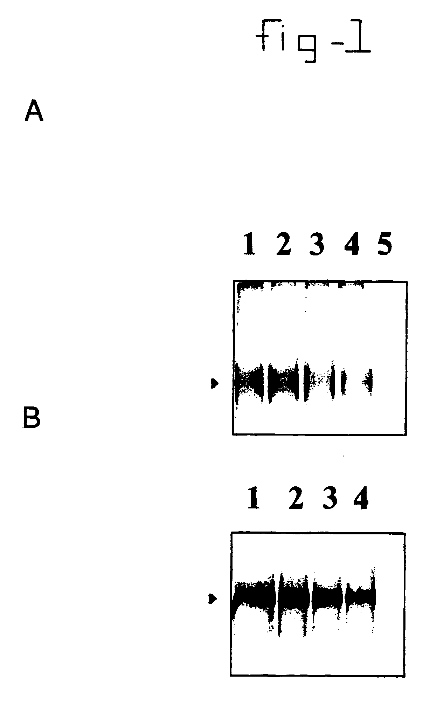

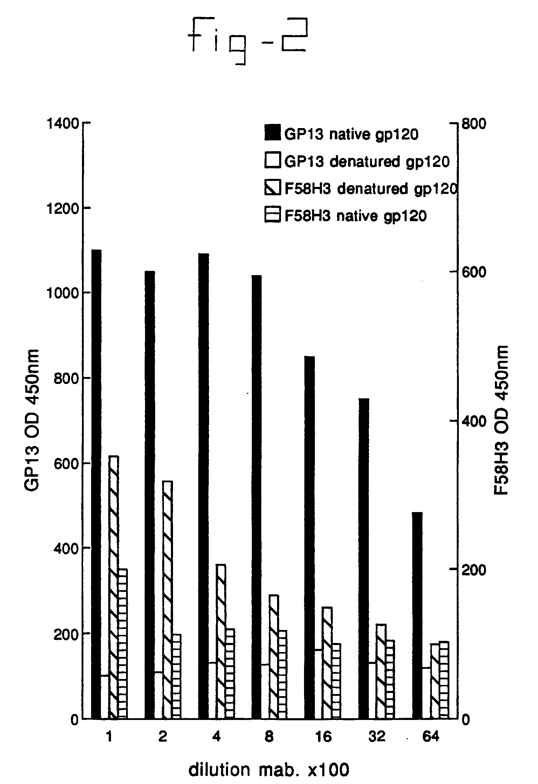

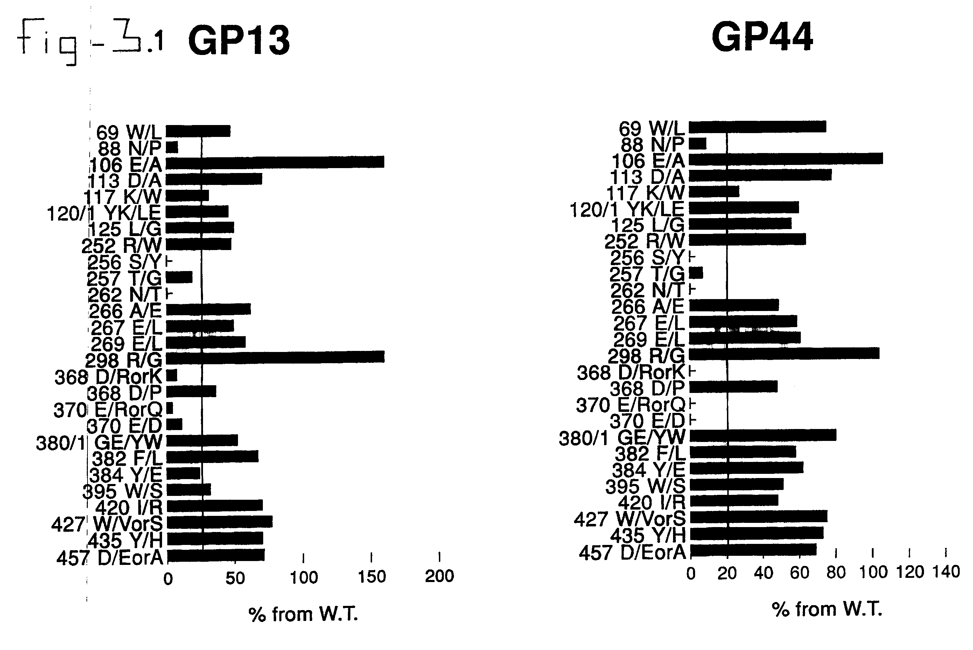

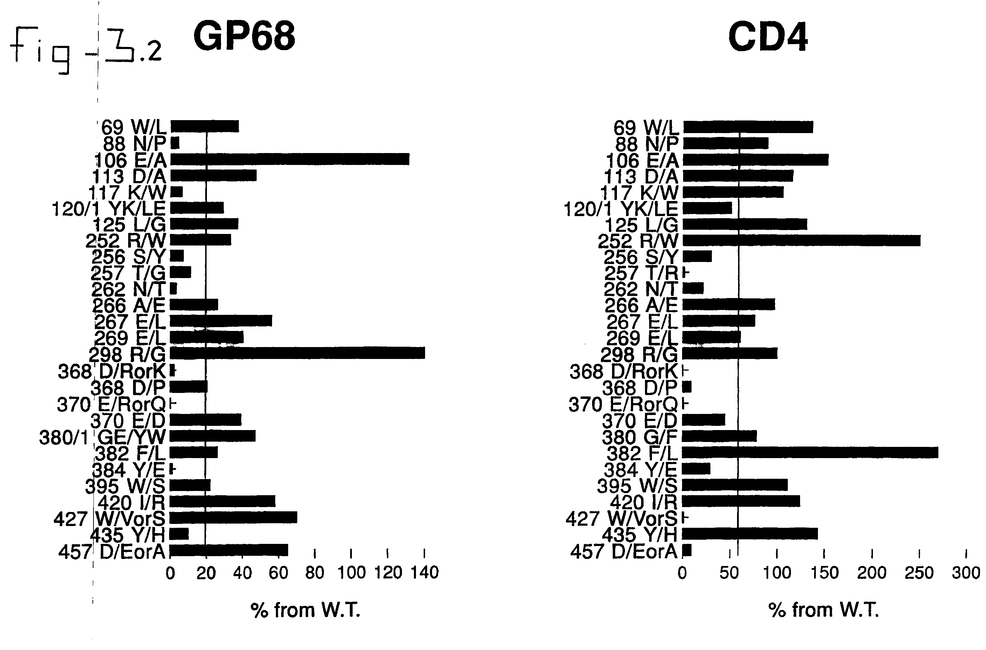

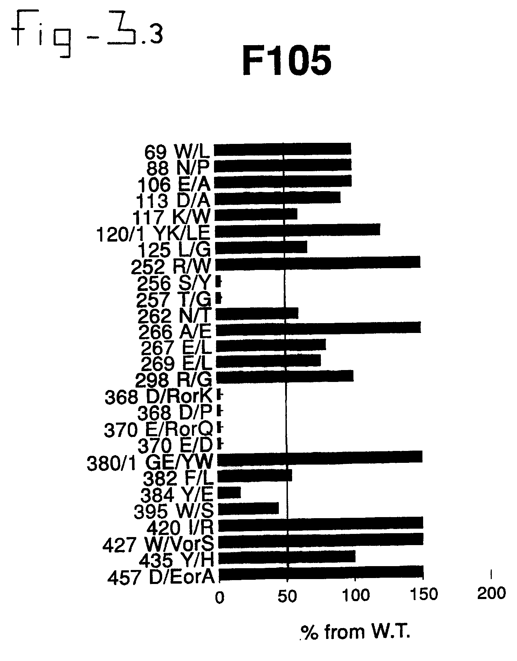

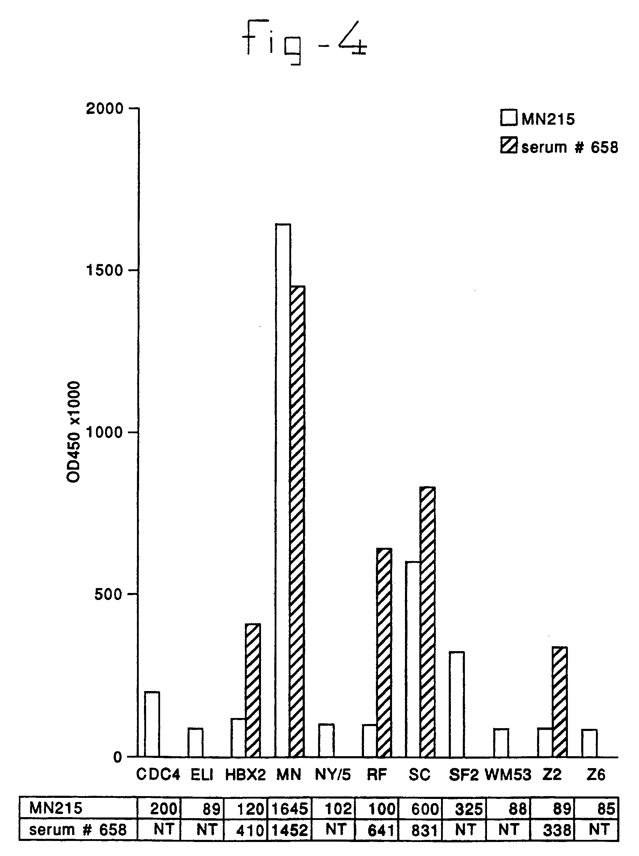

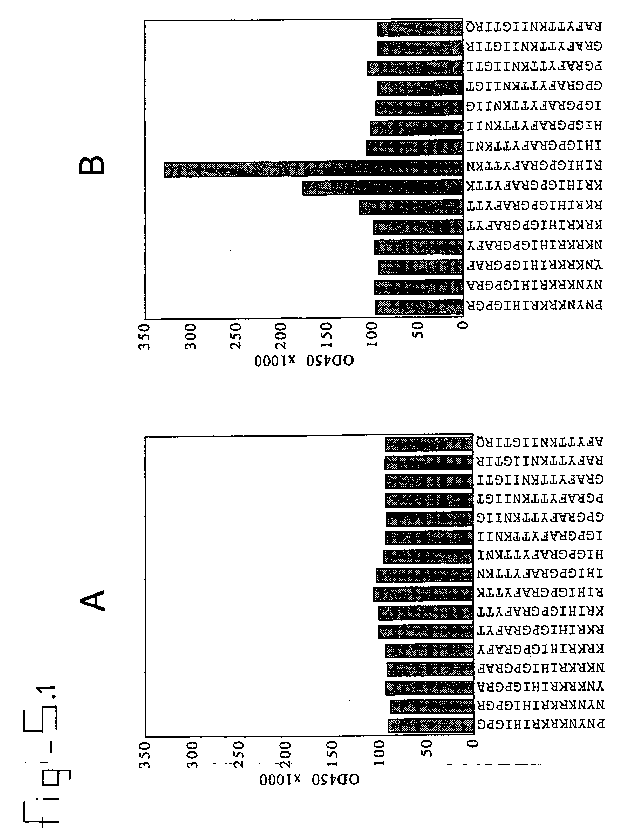

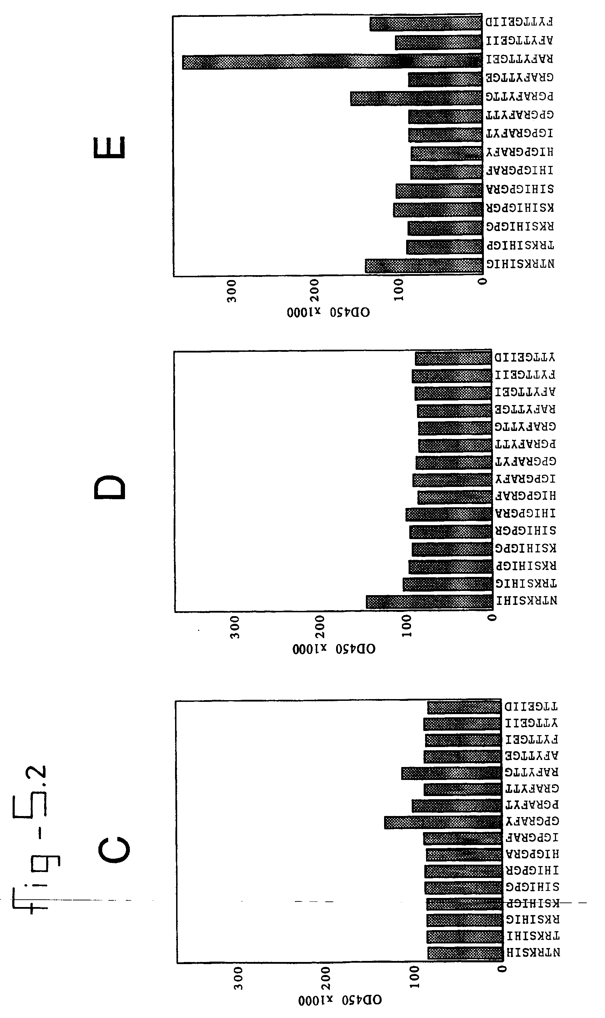

The specificity of the three human mabs for gp120 was further confirmed in a RIPA, using recombinant vaccinia viruses expressing the envelope gene of a non syncytium inducing and a syncytium inducing ( N.S.I. and S.I.) HIV-1 isolate from two Dutch individuals. As shown in figure 1, all three human mabs precipitated gp120 of both HIV-1 isolates. Precipitation of gp120 from both N.S.I. and S.I. isolates from the second donor was essentially the same. Under mild denaturing conditions only about 50% of the total gp120, as determined by precipitation of gp120 by the V3 loop specific Human mab MN215 in combination with a gp120 specific polyclonal sheep serum, was precipitated by these three human mabs. This finding together with the lack of reactivity of the three human mabs in a western blotting analysis with gp120/gp160 under denaturing conditions indicated that the antigenic site recognized by these three human mabs is conformation dependent. This was confirmed by testing the reactivity of the three human mabs and the V3 loop specific Momab F58H3 in an ELISA using a nitrocellulose solid phase. As shown in figure 2, human mab GP13 only recognized HIV-1 gp120 in its native form and not in a denatured form. The latter was recognized by F58H3, as previously described (Broliden *et al*. 1992). Human mabs GP44 and GP68 gave essentially the same results in this assay.

The broadest HIV-1 neutralizing capacity was observed with Human mab GP13 which neutralized all the HIV-1 strains tested. The concentrations of antibody needed to neutralize 1000 TCID50 of the different virus strains, ranged from 0.1 $\mu$g/ml for HIV-1 SF2 to 90 $\mu$g/ml for HIV-1 RF. Also human mab GP68 exhibited a broad VN activity, and only failed to neutralize HIV-1 RF at antibody concentrations tested (<250 $\mu$g/ml). Human mab GP44 only neutralized HIV-1 SF2 under these conditions at levels $\geq$ 60 $\mu$g/ml (table 4). These data indicate collectively that the three broadly reactive VN human mabs recognize a highly conserved antigenic site of HIV-1 that is probably associated with the CD4 binding site of gp120.

The reactivity in ELISA of these three human mabs with the panel of HIV-1 gp120 mutant glycoproteins, containing single aa substitutions (Olshevsky *et al*. 1991), are shown in figure 3. The reactivities with the mutant proteins are plotted as percentages of their reactivities with the wild type (HXBc2) gp120. For comparison, the reactivities of these mutant glycoproteins with sCD4 and F105, a previously described Human mab of similar specificity, are also shown in figure 3 (Olshevsky *et al*. 1990; Thali *et al*. 1991). Changes at aa 256, 257, 368 and 370 abrogate the activity of all human mabs of this specificity and CD4, indicating that these aa are at the core of this antigenic site. It is important to note that change in aa 256 leads to more than 60% reduction in gp160 precursor processing. Change in aa 262 which leads to a reduced cell association of the protein, also leads to a reduced recognition by our three human mabs and CD4, but not by human mab F105. Change in aa 88 which is a glycosylation site of gp120 (Modrow *et al.* 1987) proved associated with loss of reactivity of all the three newly developed human mabs of this specificity, but not of CD4 or human mab F105. Changes in aa 117 and 435 proved uniquely associated with the loss of recognition by human mab GP68.

**Specificity of Human mab MN215**

Human mab MN215, which did not inhibit the binding of human mabs GP13 and GP68 to HIV-1 IIIB gp120 (table 2), and did not react in ELISA with baculo recombinant HIV-1 IIIB gp120 or HIV-1 IIIB gp160, nor with the other lentivirus glycoproteins or V3 loop peptide of HIV-1 IIIB (table 1), also failed to neutralize HIV-1 IIIB. It did however neutralize HIV-1 MN and HIV-1 SF2 (table 4). When this human mab was tested with a panel of V3 loop peptides representing the V3 loop of several antigenically distinct HIV-1 strains, the highest reactivity was observed with the V3 loop peptide of HIV-1 MN (figure 4). Some reactivity was also observed with the V3 loop peptides of HIV-1 SC and HIV-1 SF2. Interestingly also the serum of donor #658,taken at the time of PBMC collection, showed the highest reactivity with the HIV-1 MN peptide (figure 4).

To determine the core and the extent of the epitope, human mab MN215 was tested in a pepscan for its reactivity with HIV-1 MN V3 loop peptides, overlapping by all but one aa and varying in length from 8 to 16 aa (Meyers *et al.* 1989). A V3 loop sequence (NTRKSIHIGPGRAFYTTGEIID) (SEQ ID NO 2) more related to the V3 loop sequence found in serum from donor #658 taken at seroconversion (TRKGIHIGPGRYTTGEIIGDIRQAH) (SEQ ID NO 3) was used in the second experiment. This sequence is also more related to sequences found in Dutch virus isolates (Groenink *et al.* 1991). When the first sequence was used, the shortest peptide recognized was 15 aa in length and the recognized sequence overlapped with the GPG $\beta$-turn, from which it extended in both the C- and N-terminal directions. When the Dutch HIV-1 MN like sequence was used in this pepscan, the shortest peptide recognized was 9 aa in length, overlapped with the GPG $\beta$-turn, from which extended in C-terminal direction (figure 5b). The minimal epitope in the second experiment has a 100% homology with the sequence found in the serum of the donor #658.

Table 1

Table 1 shows midpoint titers of protein A purified human mabs in different ELISAs. Binding of antigen to the ELISA plate was confirmed with monoclonal antibodies (HIV-1 gp120, HIV-1 gp160 and V3 IIIB) or polyclonal human, cat and chimpanzee (HIV-2, SIV-mac glycoprotein, FIV gp160, V3 loop MN) serum antibodies directed against the antigen coat.

|  | GP13 (90µg/ml) | GP44 (180µg/ml) | GP68 (270µg/ml) | MN215 (60µg/ml) |
|---|---|---|---|---|
| HIV-1 gp160 | 4800 | 280 | 12000 | <10 |
| HIV-1 gp120 | 128000 | 6400 | 450000 | <10 |
| HIV-2 gp105 | <10 | <10 | <10 | <10 |
| SIV-mac gp | <10 | <10 | <10 | <10 |
| FIV gp160 | <10 | <10 | <10 | <10 |
| V3 loop HIV-1 MN | <10 | <10 | <10 | 640 |
| V3 loop HlV-1 IIIB | <10 | <10 | <10 | <10 |

Table 2

Human mabs GP13 and GP68 were biotinylated and the midpoint titre of these biotinylated human mabs on the ConA rec. gp120 ELISA was determined. Binding of these biotinylated human mabs at their midpoint titre was inhibited by the human mabs and sCD4 and not by a human mab of the IgG1 subclass specific for gp41 of HIV-1 (K14). The human mabs were used in a concentration of 7 µg/ml and sCD4 at a concentration of 75 ng/well. Mean percentages of the midpoint titre maximum OD450 are shown. The assays were performed in triplicate.

Table 2

|  | GP13 | GP44 | GP68 | MN215 | K14 | sCD4 |
|---|---|---|---|---|---|---|
| 1171GP 13°biot | 58 | 58 | 10 | <5 | <5 | 42 |
| GP68 °biot | 43 | 39 | 37 | <5 | <5 | 31 |

Table 3

Recognition of HIV strains in immunostaining by human mabs GP13, GP68 and GP44. N.T. is not tested

| HIV isolate (HIV-1/HIV-2, origin) | GP13 | GP68 | GP44 |
|---|---|---|---|
| MN            (HIV-1, USA) | + | + | + |
| IIIB          (HIV-1, Europe) | + | + | + |
| RF            (HIV-1, Haiti) | + | + | + |
| NY/5;LAV-1 (HIV-1, Europe) | + | + | N.T. |
| SF2           (HIV-1, USA) | + | + | + |
| CBL-4         (HIV-1, Tanzania) | + | + | + |
| Z34           (HIV-1, Zaire) | + | + | N.T. |
| Z84           (HIV-1, Zaire) | + | + | N.T. |
| Z129          (HIV-1, Zaire) | + | + | N.T. |
| GL-1          (HIV-1, Zaire) | + | + | N.T. |
| GL-3          (HIV-1, Zaire) | + | + | N.T. |
| U455          (HIV-1, Uganda) | + | + | N.T. |
| LAV-2         (HIV-2, West Africa) | - | - | N.T. |
| CBL-22        (HIV-2, Gambia) | - | - | - |

<u>Table 4</u>

In vitro VN activity given as the lowest concentration of human mab in
μg/ml neutralizing 1000 TCID50 in a syncytium inhibition assay.

Human Monoclonal Antibody

| virus strain | GP13 | GP68 | GP44 | MN215 |
|---|---|---|---|---|
| HIV-1 SF2 | 0.1 | 0.7 | 60 | <70 |
| HIV-1 MN | 30 | 11 | >240 | 12.5 |
| HIV-1 I11B | 45 | 30 | >240 | >250 |
| HIV-1 RF | 90 | >250 | >240 | N.T. |
| HIV-1 CBL4 | 12 | 11 | >240 | N.T. |
| HIV-1 Z34 | 8 | 11 | >240 | N.T. |
| HIV-2 ROD | >250 | >250 | >240 | N.T. |
| HIV-2 CBL-22 | >250 | >250 | >240 | N.T. |

Description of the figures:

Figure 1
Figure 1A: A qualitative RIPA with human mabs GP13 (lane I), GP44 (lane II), GP68 (lane III), MN215 (lane IV) and K14 (lane V) on supernatant of the N.S.I. recombinant vaccinia HIV-1 envelope under non denaturing conditions. Figure 1B: Precipitation of the supernatant of the S.I. recombinant vaccinia envelope with human mabs MN215 (lane II), GP13 (lane III), GP68 (lane IV) and K14 (lane V) and in lane I the precipitation with polyclonal sheep anti gp120 is given. The precipitation of the S.I. recombinant vaccinia HIV-1 envelope was performed under mild denaturing conditions.
Figure 2
Mean $OD_{450}$ values obtained from three individual tests with humab GP13 and F58H3 in ELISA with denatured and non denatured gp120. Binding of gp120 to the nitrocellulose bottom of the well was determined with pooled serum of HIV-1 seropositive individuals and in both cases (non denatured and denatured) found positive (data not shown).
Figure 3
Binding ability of the human mabs and of CD4 to mutant gp120 glycoproteins (vertical axis) compared to binding to wild type gp120 (% from wild type, horizontal axis)
Figure 4
Specificity in ELISA of human mab MN215 and serum from donor # 658 on a panel of 20-22 aa long peptides comprising the V3 loop of different HIV-1 strains from different geographical areas. For both analysis Goat anti human IgG HRP labeled was used to detect bound antibody.
Figure 5
Pepscan analysis of MN215 with HIV-1 MN sequence (A,B) and a V3 loop sequence more related to Dutch HIV-1 sequences (A. Andeweg *et al.* 1992; Groenink *et al.* 1991) (C,D,E). Concentration of human mab used was approximately 0.7 μg/ml. Background OD450 for both analysis was 0.095 (standard deviation 0.001). Given are the 14 (A) and 15 (B) aa long sets of peptides for the MN sequence (from SEQ ID NO 1) and for the other sequence the 7 (C), 8 (D) and 9 (E) aa long peptides (from SEQ ID NO 2) are given.

Literature references

Andeweg, A.C., Groenink, M., Leeflang, P., de Goede, R.E.Y., Osterhaus, A.D.M.E., Tersmette, M., Bosch, M.L. (1992). Genetic and functional analysis of a set of Human Immunodeficiency Virus type 1 envelop genes obtained from biological clones with varying syncytium inducing capacities. Submitted.

Ascher, M.S. and Sheppard, H.W. (1988). AIDS as immune system activation: A model for pathogenesis. *Clin. Exp. Immunol.* **73**, 165-167.

Bradford, M.M. (1976). A rapid and sensitive method for the quantitation of microgram quantities of protein utilizing the principle of protein-dye binding. *Anal. Biochem.* **72**, 248-254.

Broliden, P.A., Vongegerfelt, A., Clapham, P., Rosen, J., Fenyo, E.M., Wahren, B., and Broliden, K. (1992). Identification of Human Neutralization-Inducing Regions of the Human Immunodeficiency Virus Type-1 Envelope Glycoproteins. Proc. Natl. Acad. Sci. USA **89**, 461-465.

Cordell, J., Moore, J.P., Dean, C.J., Klasse, P.J., Weiss, R.A., and McKeating, J.A. (1991). Rat Monoclonal Antibodies to Nonoverlapping Epitopes of Human Immunodeficiency Virus Type-1 gp120 Block CD4 Binding In vitro. *Virology* **185**, 72-79.

Dowbenko, D., Nakamura, G., Fennie, C., Shimasaki, C., Riddle, L., Harris, R., Gregory, T., and Lasky, L. (1988). Epitope mapping of the human immunodeficiency virus type 1 gp120 with monoclonal antibodies. *J. Virol.* **62**, 4703-4711.

Emini, E.A., Schleif, W.A., Nunberg, J.H., Conley, A.J., Eda, Y., Tokiyoshi, S., Putney, S.D., Matsushita, S., Cobb, K.E., Jett, C.M., Eichberg, J.W., and Murthy, K.K. (1992). Prevention of HIV-1 Infection in Chimpazees by gp120 V3 Domain-Specific Monoclonal Antibody. *Nature* **355**, 728-730.

Fung, M.S.C., Sun, C.R.Y., Gordon, W.L., Liou, R.S., Chang, T.W., Sun, W.N.C., Daar, E.S., and Ho, D.D. (1992). Identification ad Characterization of a Neutralization Site Within the Second Variable Region of Human Immunodeficiency Virus Type-1 gp120. *J. Virol.* **66**, 848-856.

Geyssen, H.M., Meloen, R.H., and Barteling, S. J. (1984). Use of peptide synthesis to probe viral antigens for epitopes to a resolution of a single aminoacid. *Proc. Natl. Acad. Sci. USA* **81**, 3998-4002.

Gorny, M. K., Xu, J.Y., Gianakakos, V., Karwowska, S., Wiliams, C., Sheppard, H.W., Hanson, C.V., Zolla-Pazner, S. (1991). Production of site-selected neutralizing human monoclonal antibodies against the third variable domain of the human immunodeficiency virus type 1 envelope glycoprotein. P*roc. Natl. Acad. Sci. USA*. **88**, 3238-3242.

Goudsmit, J., Boucher, C.A.B., Meloen, R.H., Epstein, L.G., Smit, L., van der Hoek, L., and Bakker, M. (1988a). Human antibody response to a strain-specific HIV-1 gp120 epitope associated with cell fusion inhibition. *AIDS.* **2**,157-64.

Goudsmit, J., Debouck, C., Meloen, R.H., Smit, L., Bakker, M., Asher, D.M., Wolff, A.V., Gibbs, C.J.,Jr., and Gajdusek, D.C. (1988b). Human immunodeficiency virus type 1 neutralization epitope with conserved architecture elicits early type-specific antibodies in experimentally infected chimpanzees. *Proc. Natl. Acad. Sci. USA*. **85**,4478-4482.

Groenink, M., Fouchier, R. A. M., De Goede, R. E. Y., de Wolf, F., Gruters, R. A., Cuypers, H. T. M., Huisman, H. G., and Tersmette, M. (1991). Phenotypic heterogeneity in a panel of infectious molecular human immunodeficiency virus type 1 clones derived from a single individual. *J. Virol.* **65**, 1968-1975.

Ho, D.D., Fung, M.S.C., Cao, Y., Li, X.L., Sun, C., Chang, T.W., Sun, N.C. (1991). Another discontinuous epitope on glycoprotein gp120 that is important in human immunodeficiency virus type 1 neutralization is identified by a monoclonal antibody. *Proc. Natl. Acad. Sci. USA.* **88**, 8949-8952.

Kang, C.Y., Nara, P., Chamat, S., Caralli, V., Ryskamp, T., Haigwood, N.L., Newman, R., and Kohler, H- (1991). Evidence for non-V3 specific neutralizing antibodies that interfere with gp120/CD4 binding in human immunodeficiency virus 1-infected humans**.** *Proc. Natl. Acad. Sci. USA.* **88**, 6171-6175.

Karpas, A., Hewlett, I.K., Hill, F., Gray, J., Byron, N., Gilgen, D., Bally, V., Oates, J.K., Gazzard, B., and Epstein, J.E. (1990). Polymerase chain reaction evidence for human immunodeficiency virus 1 neutralization by passive immunization in patients with AIDS and AIDS-related complex. *Proc. Natl. Acad. Sci. USA* **87**

Laemmli, E.K.(1970). Cleavage of structural proteins during the assembly of the head of bacteriophage T4. *Nature* **227**, 680-685.

Laman, J.D., Schellekens, M.M., Abacioglu, Y.H., Lewis, G.K., Tersmette, M., Fouchier, R.A.M., Langedijk, J.P.M., Claassen, E., and Boersma, W.J.A. (1992). Variant-Specific Monoclonal and Group-Specific Polyclonal Human Immunodeficiency Virus Type-1 Neutralizing Antibodies Raised with Synthetic Peptides from the gp120 Third Variable Domain. *J. Virol.* **66**, 1823-1831.

Langedijk, J.P.M., Back, N.K.T., Durda, P.J., Goudsmit, J., and Meloen, R.H. (1991). Neutralizing Activity of Anti-Peptide Antibodies Against the Principal Neutralization Domain of Human Immunodeficiency Virus Type-1. *J. Gen. Virol.* **72**, 2519-2526.

LaRosa, G.J., Davide, J.P., Weinhold, K., Waterbury, J.A., Profy, A.T., Lewis, J.A., Langlois, A.J., Dreesman, G.R., Boswell, R.N., Shadduck, P., Holley, L.H., Karplus, M., Bolognesi, D.P., Matthews, T.J., Emini, E.A., and Putney, S.D. (1990). Conserved sequence and structural elements in the HIV-1 principal neutralizing determinant. *Science* **249**, 932-935.

Lasky, L.A., Nakamura, G.M., Smith, D.H., Fennie, C., Shimasaki, C., Patzer, E., Berman, P., Gregory, T., Capon, D.J. (1987). Delineation of a region of the human immunodeficiency virus type gp120 glycoprotein critical for iteraction with the CD4 receptor. *Cell* **50**, 975-985.

Meyers, G., Josephs, S.F., Berzofsky, J.A., Rabson, A.B., Smith, T.F., Wong-Staal, F. (1989). *Human retroviruses and AIDS*, Los Alamos National Laboratories, Los Alamos, N.Mex. .

Modrow, S., Hahn, B.H., Shaw, G.M., Gallo, R.C., Wong-Staal, F., Wolf, H. (1987). Computer-assisted analysis of envelope protein sequences of seven Human Immunodeficiency Virus isolates: prediction of antigenic epitopes in conserved and variable regions. *J. Virol.* **61**, 570-578.

Moore, J.P., Wallace, L.A., Follett, E.A.C., and McKeating, J.A. (1989). An enzyme-linked immunosorbent assay for antibodies to the envelope glycoproteins of divergent strains of HIV-1. *AIDS* **3**, 155-163.

Moore, J.P., McKeating, J.A., Jones, I.M., Stephens, P.E., Clements, G., Thomson, S., and Weiss, R.A. (1990). Characterization of recombinant gp120 and gp160 from HIV-1: Binding to monoclonal antibodies and soluble CD4. *AIDS* **4**, 307-315.

Ohno, T., Terada, M., Yoneda, Y., Shea, K.W., Chambers, R.F., Stroka, D.M., Nakamura, M., and Kufe, D.W. (1991). A Broadly Neutralizing Monoclonal Antibody That Recognizes the V3 Region of Human Immunodeficiency Virus Type-1 Glycoprotein gp120. *Proc. Natl. Acad. Sci. USA* **88**, 10726-10729.

Olshevsky, U., Helseth, E., Furman, F. Li, J., Haseltine, W., and Sodroski, J. (1990). Identification of individual HIV-1 gp120 amino acids important for CD4 receptor binding. *J. Virol.* **64**. 5701-5707.

Posner, M.R., Hideshima, T,, Cannon, T., Mukherjee, M., Mayer, K.H., and Byrn, R.A. (1991). An IgG human monoclonal antibody that reacts with HIV-1/gp120, inhibits virus binding to cells, and neutralizes infection. *J. Immunol.* **146**, 4325-4332.

Prince, A.M., Reesink, H., Pascual, D., Horowitz, B., Hewlett, I., Murthy, K.K., Cobb, K.E., and Eichberg, J.W. (1991). Prevention of HIV Infection by Passive Immunization with HIV Immunoglobulin. *AIDS Res. Hum. Retroviruses* **7**, 971-973.

Putkonen, P., Thorstensson, R., Ghavamzadeh, L., Albert, J., Hild, K., Biberfeld, G., and Norrby, E. (1992). Prevention of HIV-2 and SIV-sm infection by passive immunization in cynomolgus monkeys. *Nature* **352**, 436-438.

Robinson, J.E., Holton, D., Pacheco Morell, S,, Liu, J., and McMurdo, H. (1990). Identification of conserved and variant epitopes of human immunodeficiency virus type 1 (HIV-1) gp120 by human monoclonal antibodies produced by EBV-transformed cell lines. *AIDS Res. Hum. Retroviruses* **6** 567-579

Robinson, W.E.,Jr., Gorny, M. K., Xu Jian Yin, Mitchell, W.M., and Zolla Pazner, S. (1991). Two immunodominant domains of gp41 bind antibodies which enhance human immunodeficiency virus type 1 infection in vitro. *J. Virol.* **65**, 4169-4176.

Siebelink, K.H.J., Chu, I-H., Rimmelzwaan, G.F., Weijer, K., Osterhaus, A.D.M.E., and Bosch, M.L. (1992). Isolation and partial characterization of infectious molecular clones of feline immunodeficiency virus obtained directly from bone marrow DNA of a naturally infected cat. *J. Virol.* **66**, 1091-1097.

Steinitz, M., Klein, G., Koskimies, S., and Makela, O. (1977). EB virus-induced B-lymphocyte cell lines producing specific antibody. *Nature* **269**, 420.

Steimer, K.S., Scandella, C.J., Skiles, P.V., Haigwood, N.L. (1991). Neutralization of divergent HIV-1 isolates by conformation dependent human antibodies to gp120. *Science* **254**, 105-108.

Thali, M., Olshevsky, U., Furman, C., Gabuzda, D., Posner, M., Sodroski, J. (1991). Characterization of a discontinuous human immunodeficiency virus type 1 gp120 epitope recognized by a broadly reactive neutralizing human monoclonal antibody. *J. Virol.* **65**, 6188-6193.

Teeuwsen, V.J.P., Siebelink, K.H.J., Crush Stanton, S., Swerdlow, B., Schalken, J.J., Goudsmit, J., van de Akker, R., Stukart, M.J., Uytde-Haag, F.G.C.M., and Osterhaus, A.D.M.E. (1990). Production and characterization of a human monoclonal antibody reactive with a conserved epitope on gp41 of human immunodeficiency virus type I. *AIDS Res. Hum. Retroviruses* **6**, 381-392.

Wysocki, L.J. and Sato, V.L. (1980). Depletion of lymphocyte subsets by panning. *Proc. Natl. Acad. Sci. USA* **75**, 2844-2848.

**EP 0 581 353 A1**

ANNEX (1/10)

SEQUENCE LISTINGS

(1)  GENERAL INFORMATION:

   (i)    APPLICANT:
       (A) NAME: De Staat der Nederlanden vertegenwoordigd door
                de Minister van Welzijn, Volksgezondheid en Cultuur
       (B) P.O. BOX: 5406
       (C) CITY: Rijswijk
       (E) COUNTRY: The Netherlands
       (F) POSTAL CODE: 2280
       (G) TELEPHONE: 70-3407911

   (ii)  TITLE OF INVENTION: Monoclonal antibodies to HIV-1 gp120

   (iii) NUMBER OF SEQUENCES: 9

   (iv) COMPUTER-READABLE FORM:
       (A) MEDIUM TYPE: Diskette
       (B) COMPUTER: IBM-compatible
       (C) OPERATING SYSTEM: MS-DOS
       (D) SOFTWARE: WordPerfect 5.1

   (v)   CURRENT APPLICATION DATA:
       (A) APPLICATION NUMBER: EP 93201959.9

(2) INFORMATION FOR SEQ ID NO: 1:

   (i) SEQUENCE CHARACTERISTCS
       (A) LENGTH: 29 aminoacids
       (B) TYPE: polypeptide

   (vi) ORIGINAL SOURCE:
       (A) ORGANISM: HIV-1

   (xi) SEQUENCE DESCRIPTION: SEQ ID NO: 1:

Pro Asn Tyr Asn Lys Arg Lys Arg Ile His Ile Gly Pro Gly Arg
1            5               10              15

AIa Phe Tyr Thr Thr Lys Asn Ile Ile Gly Thr Ile Arg Gln
              20            25         29

ANNEX (2/10)

(2) INFORMATION FOR SEQ ID NO: 2:

    (i) SEQUENCE CHARACTERISTCS
        (A) LENGTH: 22 aminoacids
        (B) TYPE: polypeptide

    (vi) ORIGINAL SOURCE:
        (A) ORGANISM: HIV-1

    (xi) SEQUENCE DESCRIPTION: SEQ ID NO: 2

```
Asn Thr Arg Lys Ser Ile His Ile GIy Pro Gly Arg AIa Phe Tyr
 1               5                   10                      15

Thr Thr Gly Glu Ile Ile Asp
                20
```

(2) INFORMATION FOR SEQ ID NO: 3:

    (i) SEQUENCE CHARACTERISTCS
        (A) LENGTH: 25 aminoacids
        (B) TYPE: polypeptide

    (vi) ORIGINAL SOURCE:
        (A) ORGANISM: HIV-1

    (xi) SEQUENCE DESCRIPTION: SEQ ID NO: 3:

```
Thr Arg Lys Gly Ile His Ile Gly Pro Gly Arg Tyr Thr Thr Gly
 1               5                   10                      15

Glu Ile Ile Gly Asp Ile Arg Gln Ala His
                20                  25
```

(2) INFORMATION FOR SEQ ID NO: 4

    (i) SEQUENCE CHARACTERISTCS
        (A) LENGTH: 342 base pairs
        (B) TYPE: nucleic acid
        (C) STRANDEDNESS: single
        (D) TOPOLOGY: linear

    (ii) MOLECULE TYPE: cDNA

    (v) FRAGMENT TYPE: internal

ANNEX (3/10)

(vi) ORIGINAL SOURCE:
    (A) ORGANISM: Homo sapiens
        GP13 light chain (V$_\kappa$IV)

(ix) FEATURE:
    (A) KEY: Complementarity Determining Region I
    (B) LOCATION: bases 70..120 (aminoacids 24-40)

(ix) FEATURE:
    (A) KEY: Complementarity Determining Region II
    (B) LOCATION: bases 166..186 (aminoacids 56-62)

(ix) FEATURE:
    (A) KEY: Complementarity Determining Region IIl
    (B) LOCATION: bases 283..306 (aminoacids 95-102)

(ix) FEATURE:
    (A) KEY: Framework IV (Jκ2 segment)
    (B) LOCATION: bases 307..342 (aminoacids 103-114)

(xi) SEQUENCE DESCRIPTION: SEQ ID NO: 4:

```
GAC ATC GTG ATG ACC CAG TCT CCA GAC TCC CTG GCT GTG TCT CTG GGC    48
Asp IIe Val Met Thr Gln Ser Pro Asp Ser Leu Ala Val Ser Leu Gly
 1               5               10                  15

GAG AGG GCC ACC ATG CAC TGC AAG TCC AGC CAG AGT GTT ATG CAC ACC    96
Glu Arg Ala Thr Met His Cys Lys Ser Ser Gln Ser Val Met His Thr
             20                  25                  30

TCC AAC CAA AGG AAC TAC TTA GCG TGG TAC CAG CAG AGA CCA GGA CAG   144
Ser Asn Gln Arg Asn Tyr Leu Ala Trp Tyr Gln Gln Arg Pro Gly Gln
         35                  40                  45

TCT CCT AGG CTG CTC ATT TAC TGG GCA TCT ACC CGG GAA TCT GGG GTC   192
Ser Pro Arg Leu Leu Ile Tyr Trp Ala Ser Thr Arg Glu Ser Gly Val
     50                  55                  60

CCT GAC CGA TTC AGT GGC AGC GGG TCT GGG ACA GAT TTC ACT CTC ACC   240
Pro Asp Arg Phe Ser Gly Ser Gly Ser Gly Thr Asp Phe Thr Leu Thr
 65                  70                  75                  80

ATC AGC AGC CTG CAG GCT GAA GAT GTG GCG GTT TAT TAC TGT CAG CAA   288
Ile Ser Ser Leu Gln Ala Glu Asp Val Ala Val Tyr Tyr Cys Gln Gln
             85                  90                  95

TAT TAT ACT ACT CCT ACG TAC ACT TTT GGC CAG GGG ACC AAG CTG GAG   336
Tyr Tyr Thr Thr Pro Thr Tyr Thr Phe Gly Gln Gly Thr Lys Leu Glu
             100                 105                 110

ATC AAA                                                            342
Ile Lys
     114
```

ANNEX (4/10)

(2) INFORMATION FOR SEQ ID NO: 5

    (i) SEQUENCE CHARACTERISTCS
        (A) LENGTH: 333 base pairs
        (B) TYPE: nucleic acid
        (C) STRANDEDNESS: single
        (D) TOPOLOGY: Iinear

    (ii) MOLECULE TYPE: cDNA

    (v) FRAGMENT TYPE: internal

    (vi) ORIGINAL SOURCE:
        (A) ORGANISM: Homo sapiens
           GP44 light chain ($V_\lambda 2$)

    (ix) FEATURE:
        (A) KEY: Complementarity Determining Region I
        (B) LOCATION: bases 67..108 (aminoacids 23-36)

    (ix) FEATURE:
        (A) KEY: Complementarity Determining Region II
        (B) LOCATION: bases 154..174 (aminoacids 52-58)

    (ix) FEATURE:
        (A) KEY: Complementarity Determining Region III
        (B) LOCATION: bases 271..294 (aminoacids 91-98)

    (ix) FEATURE:
        (A) KEY: Framework IV (J$\lambda$2 segment)
        (B) LOCATION: bases 295..333 (aminoacids 99-111)

    (xi) SEQUENCE DESCRIPTION: SEQ ID NO: 5:

```
CAG TCT GCC CTG ACT CAG CCT CCC TCC GCG TCC GGG TCT CCT GGA CAG    48
Gln Ser Ala Leu Thr Gln Pro Pro Ser Ala Ser Gly Ser Pro Gly Gln
 1           5               10              15

TCA GTC ACC ATC TCC TGC TCT GGA ACC AGC AGT GAC GTT GGT GCT TAT    96
Ser Val Thr Ile Ser Cys Ser Gly Thr Ser Ser Asp Val Gly Ala Tyr
            20              25              30

AAG TAT GTC TCC TGG TTC CAA CAA CAC CCC GGC AAA GCC CCC AAA CTC    144
Lys Tyr Val Ser Trp Phe Gln Gln His Pro Gly Lys Ala Pro Lys Leu
            35              40              45

ATG ATT TAT GAA GTC AAT GAG CGG CCC TCA GGG GTC CCT GAT CGC TTC    192
Met Ile Tyr Glu Val Asn Glu Arg Pro Ser Gly Val Pro Asp Arg Phe
         50              55              60
```

ANNEX (5/10)

```
TCT GGC TCC AAG TCT GGC AAC ACG GCC TCC CTG ACC GTC TCT GGG CTC   240
Ser Gly Ser Lys Ser Gly Asn Thr Ala Ser Leu Thr Val Ser Gly Leu
 65                      70                  75                  80

CAA CCT GAG GAT GAG GCT GAT TAT TAT TGC GCC TCA TAT GCA GGC AGT   288
Gln Pro Glu Asp Glu Ala Asp Tyr Tyr Cys Ala Ser Tyr Ala Gly Ser
                     85                  90                  95

AAC ATC GTG ATA TTC GGC GGA GGG ACA AAG TTG ACC GTC CTA GGT       333
Asn Ile Val Ile Phe Gly Gly Gly Thr Lys Leu Thr Val Leu Gly
                100                 105                 110
```

(2) INFORMATION FOR SEQ ID NO: 6

   (i) SEQUENCE CHARACTERISTCS
      (A) LENGTH: 318 base pairs
      (B) TYPE: nucleic acid
      (C) STRANDEDNESS: single
      (D) TOPOLOGY: linear

   (ii) MOLECULE TYPE: cDNA

   (v) FRAGMENT TYPE: internal

   (vi) ORIGINAL SOURCE:
      (A) ORGANISM: Homo sapiens
        GP68 light chain ($V_\kappa I$)

   (ix) FEATURE:
      (A) KEY: Complementarity Determining Region I
      (B) LOCATION: bases 70..102 (aminoacids 24-34)

   (ix) FEATURE:
      (A) KEY: Complementarity Determining Region II
      (B) LOCATION: bases 148..168 (aminoacids 50-56)

   (ix) FEATURE:
      (A) KEY: Complementarity Determining Region IIl
      (B) LOCATION: bases 265..282 (aminoacids 89-94)

   (ix) FEATURE:
      (A) KEY: Framework IV (J$\kappa$3 segment)
      (B) LOCATION: bases 283..318 (aminoacids 95-116)

   (xi) SEQUENCE DESCRIPTION: SEQ ID NO: 6:

```
GAC ATC CAG ATG ACC CAG TCT CCT TCC ACC CTG CCT GCA TCT GTA GGA    48
Asp Ile Gln Met Thr Gln Ser Pro Ser Thr Leu Pro Ala Ser Val Gly
 1               5                   10                  15
```

ANNEX (6/10)

```
GAC AGA GTC ACC ATC ACT TGC CGG GCC AGT CAG AGT ATC AGT GGA TGG    96
Asp Arg Val Thr Ile Thr Cys Arg Ala Ser Gln Ser Ile Ser Gly Trp
        20                  25                  30

CTG GCC TGG TAT CAG CAG AAA CCA GGG AAA GCC CCT AAG CTC CTG ATC   144
Leu Ala Trp Tyr Gln Gln Lys Pro Gly Lys Ala Pro Lys Leu Leu Ile
        35                  40                  45

CAT AAG ACG TCT ACT TTA GAA AGT GGG GTC CCC TCA AGG TTC AGC GGC   192
His Lys Thr Ser Thr Leu Glu Ser Gly Val Pro Ser Arg Phe Ser Gly
     50                  55                  60

AGT GGA TCT GGG ACA GAA TTC ACT CTC ACC ATC AGC AGC CTG CAG CCT   240
Ser Gly Ser Gly Thr Glu Phe Thr Leu Thr Ile Ser Ser Leu Gln Pro
 65                  70                  75                  80

GAT GAT TTC GCA ACT TAT TAT TGC CAA CAG TAT AAT AGT TTA ATT ACT   288
Asp Asp Phe Ala Thr Tyr Tyr Cys Gln Gln Tyr Asn Ser Leu Ile Thr
                85                  90                  95

TTC GGC CCT GGG ACC AAA GTG GAT ATC AAA                           318
Phe Gly Pro Gly Thr Lys Val Asp Ile Lys
            100                 105
```

(2) INFORMATION FOR SEQ ID NO: 7

    (i) SEQUENCE CHARACTERISTCS
        (A) LENGTH: 393 base pairs
        (B) TYPE: nucleic acid
        (C) STRANDEDNESS: single
        (D) TOPOLOGY: linear

    (ii) MOLECULE TYPE: cDNA

    (v) FRAGMENT TYPE: internal

    (vi) ORIGINAL SOURCE:
        (A) ORGANISM: Homo sapiens
           GP13 heavy chain ($V_HV$)

    (ix) FEATURE:
        (A) KEY: Complementarity Determining Region 1
        (B) LOCATION: bases 85..108 (aminoacids 29-34)

    (ix) FEATURE:
        (A) KEY: Complementarity Determining Region II
        (B) LOCATION: bases 142..192 (aminoacids 48-64)

ANNEX (7/10)

(ix) FEATURE:
    (A) KEY: Complementarity Determining Region III
    (B) LOCATION: bases 295..348 (aminoacids 99-116)

(ix) FEATURE:
    (A) KEY: Framework IV (JH6 segment)
    (B) LOCATION: bases 349..393 (aminoacids 117-131)

(xi) SEQUENCE DESCRIPTION: SEQ ID NO: 7:

```
GAA GTG CAG CTG GTG CAG TCC GGA GCA GAG GTG AAA AAG CCC GGG GAG    48
Glu Val Gln Leu Val Gln Ser Gly Ala Glu Val Lys Lys Pro Gly Glu
 1               5                   10                  15

TCT CTG AGG ATC TCC TGT AGG GGT TCT GGA TAC AGC TTT CCC AAT TAC    96
Ser Leu Arg Ile Ser Cys Arg Gly Ser Gly Tyr Ser Phe Pro Asn Tyr
             20                  25                  30

TGG GTC AGC TGG GTG CGC CAG ATG CCC GGG AAA GGC CTG GAG TGG ATG    144
Trp Val Ser Trp Val Arg Gln Met Pro Gly Lys Gly Leu Glu Trp Met
         35                  40                  45

GGA AAG ATT GAT CCT AGT GAC TCT GAG ACC AAC TAC AGC CCG TCC TTC    192
Gly Lys Ile Asp Pro Ser Asp Ser Glu Thr Asn Tyr Ser Pro Ser Phe
     50                  55                  60

CAA GGC CAC GTC ACC ATC TCG GCT GAC AAG TCC CTC AGT ATT GCC TAT    240
Gln Gly His Val Thr Ile Ser Ala Asp Lys Ser Leu Ser Ile Ala Tyr
 65                  70                  75                  80

CTG CAG TGG AAC AGC CTG AAG GCC TCG GAC ACC GCC ATG TAT TAC TGT    288
Leu Gln Trp Asn Ser Leu Lys Ala Ser Asp Thr Ala Met Tyr Tyr Cys
                 85                  90                  95

GCG AGA CAT AAG AGG GGC GCG CCG ACG TAT AAA GAT ATC TTG ACT GGT    336
Ala Arg His Lys Arg Gly Ala Pro Thr Tyr Lys Asp Ile Leu Thr Gly
             100                 105                 110

TAT TAT GTC GAT GGT ATG GAC GTC TGG GGC CAA GGG ACC ACG GTC ACC    384
Tyr Tyr Val Asp Gly Met Asp Val Trp Gly Gln Gly Thr Thr Val Thr
         115                 120                 125

GTC TCG TCA                                                        393
Val Ser Ser
         130
```

ANNEX (8/10)

(2) INFORMATION FOR SEQ ID NO: 8

    (i) SEQUENCE CHARACTERISTCS
        (A) LENGTH: 378 base pairs
        (B) TYPE: nucleic acid
        (C) STRANDEDNESS: single
        (D) TOPOLOGY: linear

    (ii) MOLECULE TYPE: cDNA

    (v) FRAGMENT TYPE: internal

    (vi) ORIGlNAL SOURCE:
        (A) ORGANISM: Homo sapiens
           GP44 heavy chain ($V_H1$)

    (ix) FEATURE:
        (A) KEY: Complementarity Determining Region I
        (B) LOCATION: bases 91..105 (aminoacids 31-35)

    (ix) FEATURE:
        (A) KEY: Complementarity Determining Region lI
        (B) LOCATION: bases 148..198 (aminoacids 50-66)

    (ix) FEATURE:
        (A) KEY: Complementarity Determining Region III
        (B) LOCATION: bases 295..336 (aminoacids 99-112)

    (ix) FEATURE:
        (A) KEY: Framework IV (JH4 segment)
        (B) LOCATION: bases 337..378 (aminoacids 113-126)

    (xi) SEQUENCE DESCRIPTION: SEQ ID NO: 8:

```
CAG GTG CAG CTG GTG CAG TCT GGG TCT GAG GTG AAG AAG CCT GGG GCC     48
Gln Val Gln Leu Val Gln Ser Gly Ser Glu Val Lys Lys Pro Gly Ala
 1               5                  10                  15

TCA GTG AAG GTT TCC TGC AAG GCA TCT GGA TAC ACC TTC ACC ACC TAC     96
Ser VaI Lys Val Ser Cys Lys Ala Ser Gly Tyr Thr Phe Thr Thr Tyr
                 20                  25                  30

TAT ATA CAC TGG GTG CGT CAG GCC CCT GGA CAA GGG CTT GAG TGG ATG    144
Tyr lle His Trp Val Arg Gln Ala Pro Gly Gln Gly Leu Glu Trp Met
                 35                  40                  45

GGA ATA ATC AAC CCT AGT GAT GGA AGT AGA AAC TAC GCA CAG AAC TTC    192
Gly lIe lle Asn Pro Ser Asp Gly Ser Arg Asn Tyr Ala Gln Asn Phe
          50                  55                  60

CAG GGC AGA GTC ACC ATG ACC AGG GAC ACG TCC ACG AGC ACA GTC TAC    240
Gln Gly Arg Val Thr Met Thr Arg Asp Thr Ser Thr Ser Thr Val Tyr
 65                  70                  75                  80
```

ANNEX (9/10)

```
ATG GAG CTG GGC AGA CTG AGA TCT GAA GAC ACG GCC ATA TAT TAC TGT    288
Met Glu Leu Gly Arg Leu Arg Ser Glu Asp Thr Ala Ile Tyr Tyr Cys
                85                      90                  95

GCG AGA GAT CTC CGC CTA ATG TTA TTT ACT TTG AGG GGG GGT GTG ATG    336
Ala Arg Asp Leu Arg Leu Met Leu Phe Thr Leu Arg Gly Gly Val Met
            100                     105                 110

CTT GAC TAC TGG GGC CAG GGA ACC CTG GTC ACC GTC CCC TCA           378
Leu Asp Tyr Trp Gly Gln Gly Thr Leu Val Thr Val Pro Ser
            115                 120                 125
```

(2) 1NFORMATION FOR SEQ ID NO: 9

   (i) SEQUENCE CHARACTERISTCS
      (A) LENGTH: 372 base pairs
      (B) TYPE: nucleic acid
      (C) STRANDEDNESS: single
      (D) TOPOLOGY: linear

   (ii) MOLECULE TYPE: cDNA

   (v) FRAGMENT TYPE: internal

   (vi) ORIGINAL SOURCE:
      (A) ORGANISM: Homo sapiens
        GP68 heavy chain ($V_HI$)

   (ix) FEATURE:
      (A) KEY: Complementarity Determining Region I
      (B) LOCATION: bases 91..105 (aminoacids 31-35)

   (ix) FEATURE:
      (A) KEY: Complementarity Determining Region 11
      (B) LOCATION: bases 148..198 (aminoacids 50-66)

   (ix) FEATURE:
      (A) KEY: Complementarity Determining Region III
      (B) LOCATION: bases 295..330 (aminoacids 99-110)

   (ix) FEATURE:
      (A) KEY: Framework 1V (JH4 segment)
      (B) LOCATION: bases 331..372 (aminoacids 111-124)

   (xi) SEQUENCE DESCRIPTION: SEQ ID NO: 9:

```
CAG GTG CAG CTG GTC CAG TCT GGG GCT GAA GTG AAG AAG CCT GGG TCC    48
Gln Val Gln Leu Val Gln Ser Gly Ala Glu Val lys Lys Pro Gly Ser
    1               5                   10                  15
```

ANNEX (10/10)

```
TCG GTG AAG GTC TCC TGC AAG GCT TCT GGA GGC ACA TTC AGC AGC TCA    96
Ser Val Lys Val Ser Cys Lys Ala Ser Gly Gly Thr Phe Ser Ser Ser
            20                  25                  30


ACT CTC AAC TGG GTA CGA CAG ACC CCC GGA CAA GGA CTT GAG TGG ATG   144
Thr Leu Asn Trp Val Arg Gln Thr Pro Gly Gln Gly Leu Glu Trp Met
            35                  40                  45


GGC AAA ATC ATC CCT ACC TTG GGT TCA TCA ACC TAT TCA CAG AAC TTC   192
Gly lys Ile Ile Pro Thr Leu Gly Ser Ser Thr Tyr Ser Gln Asn Phe
            50                  55                  60


CAG GGG AGA GTC ACC CTT ACC GCG GAC GAA TCC ACG AGC ACA GTC TAC   240
Gln Gly Arg Val Thr Leu Thr Ala Asp Glu Ser Thr Ser Thr Val Tyr
 65                  70                  75                  80


ATG GAA CTG AGT GGT CTG ACA TCT GCG GAC ACG GCC GTA TAT TAC TGT   288
Met Glu Leu Ser Gly Leu Thr Ser Ala Asp Thr Ala Val Tyr Tyr Cys
                85                  90                  95


GCG AAA AGT GGG GCC GGG TGG AGT AAT TTA ATC CGC TCC CCC ATT GAC   336
Ala Lys Ser Gly Ala Gly Trp Ser Asn Leu Ile Arg Ser Pro Ile Asp
            100                 105                 110


AAC TGG GGC CAG GGA ACG CTG GTC ACC GTC TCC TCA                   372
Asn Trp Gly Gln Gly Thr Leu Val Thr Val Ser Ser
            115                 120
```

## Claims

1. Monoclonal antibody to HIV-1, characterised in that it reacts with a HIV-1 gp120 glycoprotein or first variant thereof wherein the aminoacids Asn-88, Lys-117, Asn-262 and Tyr-435 are present, and exhibits an at least 50% reduced reaction with a second variant of said glycoprotein which differs from said glycoprotein or first variant thereof in that one or more of said aminoacids has been deleted or mutated.

2. Monoclonal antibody according to claim 1, wherein it reacts with said glycoprotein or first variant thereof wherein said aminoacids are present, and wherein it exhibits an at least 75% reduced reaction with a second variant of said glycoprotein comprising a mutation or deletion of aminoacids Asn-88 and/or Asn-262.

3. Monoclonal antibody according to claim 1 or 2, wherein the mutation is a substitution.

4. Monoclonal antibody according to any one of claims 1-3, which is designated GP13, GP44 or GP68.

5. Monoclonal antibody to HIV-1, comprising an aminoacid sequence in which at least five aminoacids are situated in the same relative position as the aminoacid sequences represented in any one of the SEQ ID NO's 4-9.

6. Monoclonal antibody according to any one of claims 1-5, comprising an aminoacid sequence in which at least three aminoacids correspond to aminoacids in at least one of the complementarity determining regions identified in the aminoacid sequences represented in any one of the SEQ ID NO's 4-9.

7. Anti-idiotype antibody to an antibody according to any one of claims 1-6, or a structure based on such anti-idiotype antibody.

8. Cell line producing a human or animal monoclonal antibody according to any one of claims 1-7.

9. Preparation for passive immunotherapy comprising an antibody according to any one of claims 1-6.

10. Vaccine comprising an anti-idiotype antibody according to claim 7.

11. Nucleotide sequence encoding an aminoacid sequence of an antibody to HIV-1, comprising at least 15 consecutive nucleotides of the nucleotide sequences represented in any one of the SEQ ID NO's 4-9.

12. A method of producing a monoclonal antibody to HIV-1 comprising steps of:
a) producing cell lines which produce human or animal monoclonal antibodies;
b) screening for antibody reactivity on wild type HIV-1 glycoproteins containing gp120;
c) screening for antibody reactivity on mutant HIV-1 glycoproteins containing gp120 wherein aminoacids Asn-88, Lys-117, Asn-262 and/or Tyr-435 are deleted or mutated;
d) repeatedly cloning cell lines and screening for reactivity;
e) producing monoclonal antibody which recognizes wild-type gp120 and does not recognize gp120 mutated as in step c).

fig -1

A

B

# Fig-2

Legend:
- ■ GP13 native gp120
- □ GP13 denatured gp120
- ◩ F58H3 denatured gp120
- ▤ F58H3 native gp120

GP13 OD 450nm (left axis: 0–1400)

F58H3 OD 450nm (right axis: 0–800)

dilution mab. x100

Fig_3.1 GP13

GP44

% from W.T.

% from W.T.

Fig-3.2

**GP68**

**CD4**

% from W.T.

% from W.T.

EP 0 581 353 A1

Fig-3.3

F105

Fig - 4

| | CDC4 | ELI | HBX2 | MN | NY/5 | RF | SC | SF2 | WM53 | Z2 | Z6 |
|---|---|---|---|---|---|---|---|---|---|---|---|
| MN215 | 200 | 89 | 120 | 1645 | 102 | 100 | 600 | 325 | 88 | 89 | 85 |
| serum # 658 | NT | NT | 410 | 1452 | NT | 641 | 831 | NT | NT | 338 | NT |

Fig-5.1

fig-5.2

C

D

E

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | WO-A-9 117 764 (DANA FARBER CANCER INSTITUTE) <br> * page 3, paragraph 5 -paragraph 6 * <br> * page 5, paragraph 2 - page 6, paragraph 2 * <br> * page 8, paragraph 3 - page 10, paragraph 1 * <br> * page 11, paragraph 3 - page 12, paragraph 1 * <br> * page 20, paragraph 3 * <br> * page 23, paragraph 2 - page 28, paragraph 1 * <br> --- | 1-4, 7-10,12 | C12N5/10 <br> C12P21/08 <br> A61K39/42 |
| D,X | JOURNAL OF VIROLOGY <br> vol. 64, no. 12, 1990, BALTIMORE,USA <br> pages 5701 - 5707 <br> OLSHEVSKY ET AL 'IDENTIFICATION OF INDIVIDUAL HUMAN IMMUNODEFICIENCY VIRUS TYPE 1 GP120 AMINO ACIDS IMPORTANT FOR CD4 RECEPTOR BINDING' <br> * the whole document * <br> --- | 1-4, 7-10,12 | |
| X | WO-A-9 107 494 (XOMA CORPORATION) <br> * front page,abstract * <br> * page 2, paragraph 3 - page 5, paragraph 2 * <br> * page 14, paragraph 4 - page 18, paragraph 3; figures 1,2 * <br> --- | 5-11 | TECHNICAL FIELDS SEARCHED (Int. Cl.5 ) <br><br> C12N <br> C12P <br> C07K <br> A61K |
| P,X | EP-A-0 519 866 (CIBA-GEIGY AG) <br> * page 2, line 55 - page 3, line 22 * <br> * page 12, line 43 - page 13, line 28 * <br> * pages 33-37,'sequence listings' * <br> --- | 5-11 | |
| A | WO-A-9 113 148 (ROGER WILLIAMS GENERAL HOSPITAL) <br> * claims 1-11 * <br> --- | 1-12 | |
| | -/-- | | |

The present search report has been drawn up for all claims

| Place of search | Date of completion of the search | Examiner |
|---|---|---|
| THE HAGUE | 15 OCTOBER 1993 | SITCH W.D.C. |

EPO FORM 1503 03.82 (P0401)

European Patent
Office

**EUROPEAN SEARCH REPORT**

Application Number

EP    93 20 1959
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | DE-A-3 819 804 (ORPEGEN MEDIZINISCH-MOLEKULARBIOLOGISCHE FORSCHUNGSGESELLSCHAFT MBH) <br> * page 3, line 51 - page 4, line 10 * <br> * 'HIV ENV Peptide sequence listing' * | 1-12 | |
| | ----- | | |
| | | | TECHNICAL FIELDS SEARCHED (Int. Cl.5) |

The present search report has been drawn up for all claims

| Place of search | Date of completion of the search | Examiner |
|---|---|---|
| THE HAGUE | 15 OCTOBER 1993 | SITCH W.D.C. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P0401)